# EUROPEAN PATENT APPLICATION

(11) **EP 3 115 040 A1**
(43) Date of publication of application: **11.01.2017**
(21) Application number: 15758595.1
(22) Date of filing: 05.03.2015
(51) Int. Cl.: A61K 9/127, A61K 9/08

(54) **MICELLE CONTAINING BUBBLES FOR DRUG DELIVERY AND METHOD FOR MANUFACTURING SAME**

(30) Priority: 07.03.2014 KR 20140026922; 04.03.2015 KR 20150030387
(71) Applicant: Pacific System Co., Ltd., Incheon 402-844 (KR); Yonsei University Wonju Industry-Academic Cooperation Foundation, Gangwon-do 220-710 (KR)
(72) Inventor: SEO, Jong Bum, Wonju-si Gangwon-do 26483 (KR); WON, Jong Ho, Ansan-si Gyeonggi-do 15386 (KR); KIM, Sung Bae, Seoul 03402 (KR); SHIN, Un Chol, Namyangju-si Gyeonggi-do 12044 (KR); SONG, Gill Soo, Seoul 03454 (KR); CHA, Oh Rum, Wonju-si Gangwon-do 26494 (KR); KIM, Jin Ho, Seoul 06228 (KR)
(74) Representative: Klunker . Schmitt-Nilson . Hirsch
(86) International application number: PCT/KR2015/002111
(87) International publication number: WO 2015/133828

(57) **Abstract**

Disclosed are a micelle containing bubbles for drug delivery and a method for manufactuing the same. The micelle formed according to the present invention includes microbubbles fromed by evaporating the liquiefied inert gas due to the irradiation of ultrasonic waves to a solution in which the liquefied inert gas is mixed with a drug for disease treatment, and the micelle is formed as a unit structure in which the microbubbles are contained in a cavity and the cavity is enclosed in a shell, and delivers the drug to the human body. Therefore, the micelle can protect a material of the drug, which is delivered to the human body, as well as deliver a larger amount of the drug than the existing technique, and can be easily applied to the treatment of various diseases.

## Description

The present invention relates to a technique of drug delivery, and more particularly, to a micelle which includes a bubble capable of delivering a drug effectively to a human body and is manufactured by using cavitation caused by ultrasounds; and a method for manufacturing the micelle.

In general, a microbubble has a shell and includes gas in the shell. As it is found out in the recent studies that microbubbles have an excellent effect of enhancing contrast of ultrasonic images, studies for using such microbubbles as ultrasonic contrast agents are on a trend.

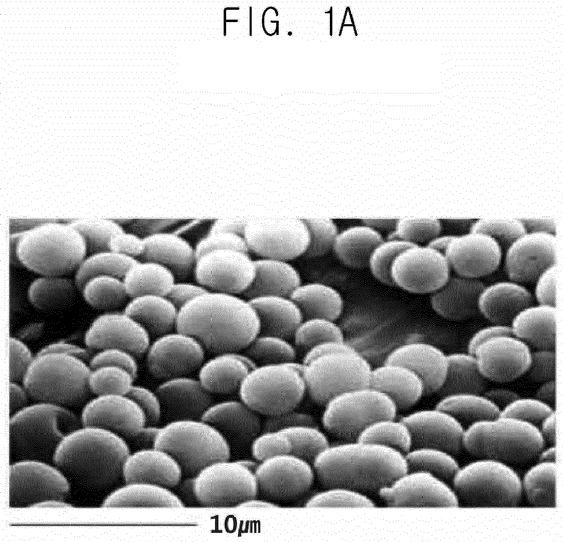

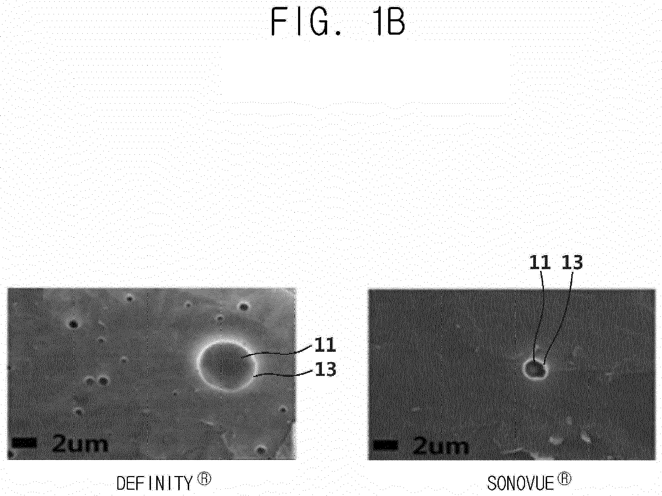

Figs. 1A and 1B are exemplary diagrams that explain conventional microbubbles used as ultrasonic contrast agents.

By referring to Fig. 1A, the microbubbles used as ultrasonic contrast agents may have an engineered shape.

More specifically, given through Cryo-TEM images, the microbubbles used as the ultrasonic contrast agents can be confirmed to be comprised of internal gas 11 and a thin shell 13 protecting the gas as shown in Fig. 1B.

In general, the gas 11 contained in the microbubble may be inert gas with a high stability. The shell 13 protecting the gas may increase the stability of the microbubble 10 and may be comprised of a variety of substances including biocompatible lipid, phospholipid, protein, etc.

However, recently, studies not only for introducing microbubbles as ultrasonic contrast agents but also for introducing them to a drug delivery system are actively conducted.

As a conventional technique, since ultrasounds can cause cavitation effectively in case of microbubbles used as ultrasonic contrast agent, efforts to make a drug be delivered to a human body are made by forming a very thin shell with the size of less than tens of nanometers (by uniformly attaching a required drug or a ligand binding substance to the shell 13 of the microbubble.

However, according to the conventional technique, since the drugs are combined with the surfaces of the shells, the drugs may be lost while the microbubbles are moved to a target position. Thus, the conventional microbubbles cannot perform a role effectively as drug carriers. In addition, the conventional microbubbles cannot load a great amount of drugs.

It is an object of the present invention to solve all the aforementioned problems.

It is another object of the present invention to provide a micelle, including a microbubble, capable of delivering drug safely to a human body efficiently.

It is still another object of the present invention to provide a method for manufacturing a micelle easily applicable to treatment of all diseases.

In accordance with one example embodiment of the present invention, a micelle includes a microbubble formed by irradiating ultrasounds to a solution into which liquefied inert gas and a drug for treatment of a disease are mixed together and evaporating the liquefied inert gas, wherein the micelle comprises: the microbubble as a form of cavity, wherein the micelle is a unit structure enclosed by a shell, and wherein the micelle delivers the drug to a human body.

In accordance with one example embodiment of the present invention, the unit structure is capsule-shaped.

In accordance with one example embodiment of the present invention, the microbubble is a space formed by the evaporation of the liquefied inert gas in the solution due to cavitation caused by the ultrasounds.

In accordance with one example embodiment of the present invention, the liquefied inert gas is perfluorobutane.

In accordance with one example embodiment of the present invention, the solution is manufactured by mixing perfluorobutane and a pre-manufactured mixture in which at least one of saline solution, glycerol, propylene glycol, and powdered phospholipid at temperature range between -15°C and 5°C and then mixing the drug for the treatment of the disease.

In accordance with one example embodiment of the present invention, the shell provides a space where the drug left in a liquid state after the evaporation of the liquefied inert gas.

In accordance with one example embodiment of the present invention, the drug loaded to the unit structure is changeable to make the micelle applicable to treatment of at least one disease.

In accordance with another example embodiment of the present invention, a method for manufacturing a micelle capable of delivering a drug, includes the steps of: manufacturing a first solution by mixing liquefied inert gas and a pre-manufactured mixture; manufacturing a second solution by mixing the drug for treatment of a disease with the first solution; and forming a microbubble in the micelle by irradiating ultrasounds to the second solution to create cavitation in the second solution.

In accordance with another example embodiment of the present invention, the pre-manufactured mixture is manufactured by mixing at least one of saline solution, glycerol, propylene glycol, and powdered phospholipid.

In accordance with another example embodiment of the present invention, at the step of manufacturing the first solution, perflouorobutane as the liquefied inert gas is mixed with the pre-manufactured mixture at temperature range between -15°C and 5°C.

In accordance with another example embodiment of the present invention, at the step of manufacturing the second solution, the micelle is applicable to treatment of at least one disease by changing the drug to be mixed with the first solution.

In accordance with another example embodiment of the present invention, at the step of forming the microbubble in the micelle, the microbubble is formed by the evaporation of the liquefied inert gas in the second solution due to cavitation caused by the ultrasounds if the ultrasounds are irradiated to the second solution and the capsule-shaped micelle, which includes the microbubble as a form of cavity and is enclosed by a shell that provides a space, where the drug left in a liquid state after the evaporation of the liquefied inert gas, is formed.

The above and other objects and features of the present invention will become apparent from the following description of preferred embodiments given in conjunction with the accompanying drawings, in which:
Figs. 1A and 1B are exemplary diagrams that explain conventional microbubbles used as ultrasonic contrast agents.
Fig. 2 is an exemplary diagram illustrating a configuration of a micelle including a bubble for drug delivery in accordance with an example embodiment of the present invention.
Fig. 3 is an exemplary diagram explaining shapes of micelles including bubbles for drug delivery in accordance with an example embodiment of the present invention.
Fig. 4 is an exemplary diagram explaining a composition ratio of a micelle including a bubble for drug delivery in accordance with an example embodiment of the present invention.
Fig. 5 is an example explaining detail components of a micelle including a bubble for drug delivery in accordance with an example embodiment of the present invention.
Figs. 6A and 6B show exemplary diagrams explaining a method for identifying a drug loaded to a micelle including a bubble for drug delivery in accordance with an example embodiment of the present invention.
Figs. 7A to 7C illustrate exemplary diagrams in which a drug loaded to a micelle including a bubble for drug delivery is identified through confocal images in accordance with an example embodiment of the present invention.
Figs. 8A to 8D depict exemplary diagrams in which a drug loaded to a micelle including a bubble for drug delivery is identified through microscopic images in accordance with an example embodiment of the present invention.
Fig. 9 is an exemplary diagram in which the drug loaded to a micelle including a bubble for drug delivery is identified through electrophoresis in accordance with an example embodiment of the present invention.
Fig. 10 is a graph representing a result identified through electrophoresis in accordance with an example embodiment of the present invention.
Fig. 11 is a flow chart explaining a method for manufacturing a micelle including a bubble for drug delivery in accordance with an example embodiment of the present invention.

The present invention may be applied to various changes and have several embodiment examples. Specific example embodiments will be illustrated in drawings and detailed explanation will be made. But it must be understood that these are not intended to limit the present invention to the specific example embodiments and include all changes, equivalents or substitutes included in the thought and technical scope of the present invention. In the drawings, like numerals refer to the same or similar functionality throughout the several views.

Terms such as first, second, A, and B may be used to explain a variety of components, but the components must not be limited by such terms. The terms are used only for the purpose of distinguishing one component from others. For example, a first component may be named as a second one without being out of the scope of the rights of the present invention and similarly, the second component may be named as the first one. A term "and/or" includes one or combination of items written in the plural forms.

When a certain component is mentioned to be "connected" or "accessed" to another one, it may be directly connected or accessed to the another component but it must be understood that there may be another component in-between. Meanwhile, when a certain component is mentioned to be "directly connected" or "directly accessed" to another one, it must be understood that there is no component in-between.

The terms used in this application are used only to explain specific example embodiments, but this is not intended to limit the present invention. Unless otherwise apparently specified in a context, a single expression includes a plural expression. In the present application, it must be understood that terms such as "include", "have" are intended to designate that there exists characteristics, numerals, steps, motions, components, parts, or their combinations in the specification but the possibility of being or adding one or more other characteristics, numerals, steps, motions, components, parts, or their combinations is not excluded.

Unless otherwise defined, all terms used herein, including technical or scientific terms, have the same meanings generally understood by a person having ordinary skill in the technical field of the present invention. The terms generally used and pre-defined must be interpreted to have the same meanings to those in the context of relevant technology, and unless clearly defined in the present application, they are not construed as having ideal or excessively formal meanings.

Detailed explanation on desirable example embodiments in accordance with the present invention will be made below by referring to the attached drawings.

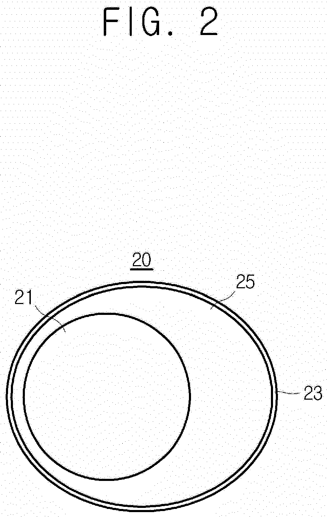

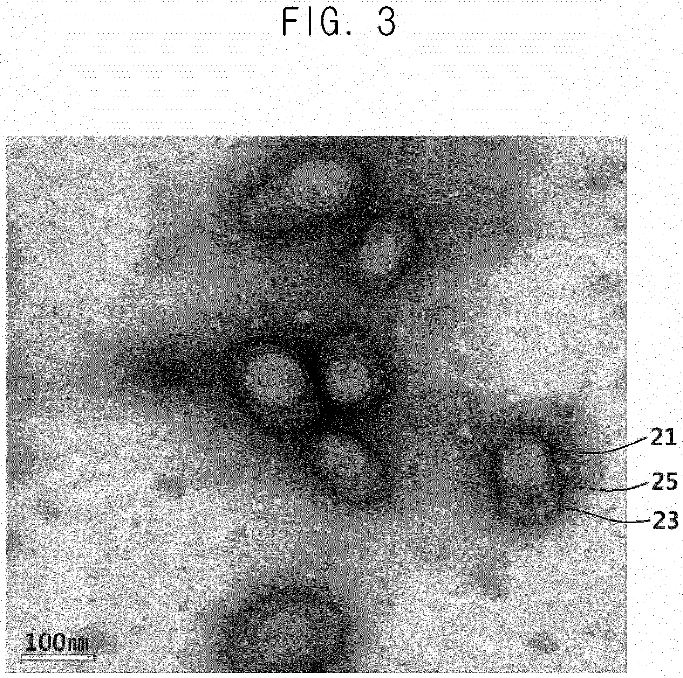

Fig. 2 is an exemplary diagram illustrating a configuration of a micelle including a bubble for drug delivery in accordance with an example embodiment of the present invention and Fig. 3 is an exemplary diagram explaining shapes of micelles including bubbles for drug delivery in accordance with an example embodiment of the present invention.

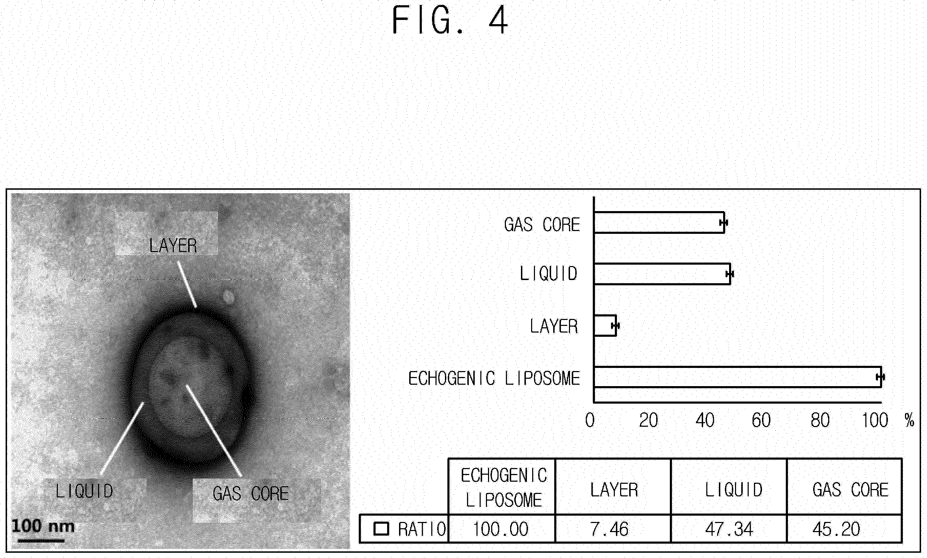

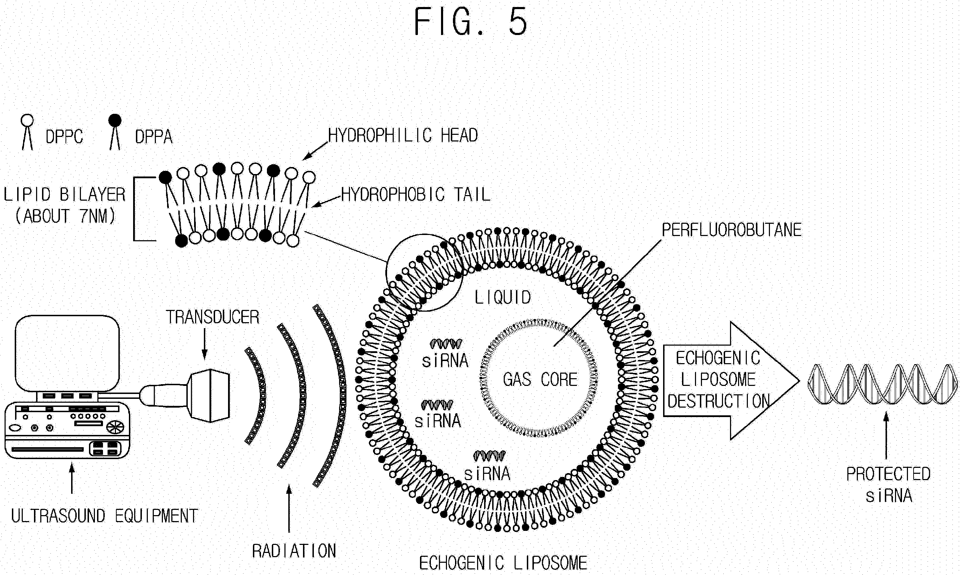

Fig. 4 is an exemplary diagram explaining a composition ratio of a micelle including a bubble for drug delivery in accordance with an example embodiment of the present invention and Fig. 5 is an example explaining detail components of a micelle including a bubble for drug delivery in accordance with an example embodiment of the present invention.

By referring to Figs. 2 to 5, a shape of a micelle 20 which includes a microbubble may be explained.

In the past, a microbubble was used as an ultrasonic contrast agent, but, recently, studies for making the microbubbles play a role for drug delivery have been actively conducted.

Therefore, a bubble was set to be formed by strongly shaking, or irradiating strong ultrasounds while gas was mixed with a shell. This causes the shell to exist between the gas and a liquid as a polar fluid to thereby create a monolayer. Herein, the monolayer includes hydrophobic parts of the shell which contact the gas and hydrophilic parts thereof which contact a solution.

To make the shell thick, as the case may be, a method for removing moisture from the shell by freezing the shell rapidly at lower pressure and then additionally attaching a substance to the shell was used. However, it was difficult to include a fluid in the shell if several procedures were not repeated.

Therefore, after a micelle was made by rapidly mixing a mixture liquid with a lipid (typically, phospholipid) or irradiating ultrasounds to a fluid and then rapidly freezing it at ultra-low pressure for the evaporation of some liquid in the micelle, the left shell after the evaporation of the liquid was made to be used again. However, a phospholipid bilayer shell may be formed, but it was difficult to allow enough amount of drug to be contained inside the micelle.

Accordingly, the present invention proposes a method for forming a micelle which includes a microbubble, a shell, and an intermediate layer between the microbubble and the shell where a drug is loaded by mixing gas at a state of liquid with a solution and then raising the temperature of the mixed solution in use of ultrasounds. Herein, the boiling point of the gas may be relatively high.

Herein, the micelle 20 may have the liquid part and the microbubble capable of delivering a drug in the liquid part to a target position. The micelle 20 in accordance with the present invention is more effective than the conventional micelle which loads a drug in its surface of a shell because the micelle 20 can load a greater amount of the drug.

The micelle 20 may include a microbubble 21, a shell 23 and a drug 25.

In detail, the micelle 20 includes the microbubble 21 with a form of cavity to deliver the drug to a human body. Further, the micelle 20 has a shape of capsule enclosed by the shell 23. Between the microbubble 21 and the shell 23, the drug 25 may be loaded for disease treatment.

First of all, the microbubble 21 may mean a space formed from the evaporation of liquefied inert gas included in a solution due to cavitation caused by ultrasounds. In detail, the ultrasounds are irradiated onto the solution, in which the liquefied inert gas and the drug for disease treatment are mixed together.

Herein, the inert gas may be perflouorobutane but it is not limited to this. This inert gas may be easily mixed with a pre-manufactured mixture because the boiling point of the perflouorobutane is - 2.56°C.

Besides, the solution is manufactured by mixing the perfluorobutane and the pre-manufactured mixture in which at least one of saline solution, glycerol, propylene glycol, and powdered phospholipid at temperature range between -15°C and 5°C and then mixing the drug for the treatment of the disease.

The reason of mixing the pre-manufactured mixture with the perflouorobutane at the temperature range between - 15°C and 5°C is that the pre-manufactured mixture may be easily mixed.

The drug 25 for the disease treatment may be included in liquid left after the liquefied inert gas is evaporated from the solution and it may be loaded between the microbubble 21 and the shell 23. Herein, since the drug mixed in the solution can be changed, the micelle 20 can deliver various kinds of drugs to various human body. Therefore, it may be applied to treatment of a variety of types of diseases.

The shell 23 may make the drug reach the human body safely by maintaining the space where the drug is loaded.

In detail, as shown in Fig.3, the micelles 20 include the microbubbles 21 which are represented as relatively brightest parts; the drugs 25 which are represented as dark parts enclosing the microbubbles 21; and the shells 23 formed outermost to keep the space where the drugs 25 are loaded. Therefore, the micelles 20 may be capsule-shaped.

For example, each of the micelles 20 may mean an echogenic liposome which can be affected by ultrasounds. The Liposome mostly has a shape of capsule such as circular or oval shape. As illustrated in Fig. 4, the microbubble 21 corresponds to gas core with a portion of 45.20% of the micelle 20, the drug 25 at the state of liquid occupies 47.34% of the micelle 20, and the shell 23 occupies 7.46% of the micelle 20.

More specifically, as shown in Fig. 5 which represents an echogenic liposome, if radiation is applied to a solution in which phospholipid such as DPPC and DPPA is mixed through a transducer of ultrasound equipment, hydrophilic parts may access the liquid and hydrophobic parts avoid the liquid. Herein, the hydrophobic parts may be assembled with one another. This may cause a bilayer structure with a thickness of approximately 7 nanometers to thereby form the shell 23.

At the time, the microbubble 21 formed by perflouorobutane and the drug 25 including siRNA effective to a gene therapy may be contained inside the shell 23. Accordingly, when the echogenic liposome is destroyed, siRNA as a drug once protected inside the shell 23 may be delivered to the human body.

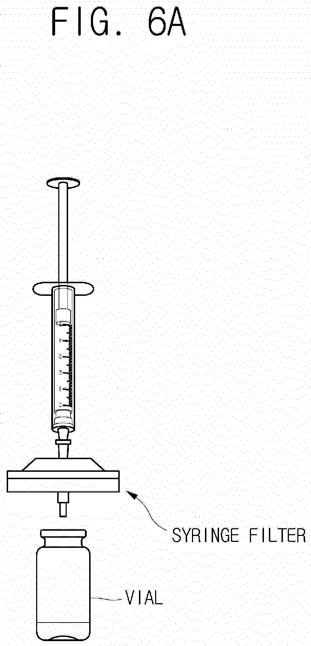

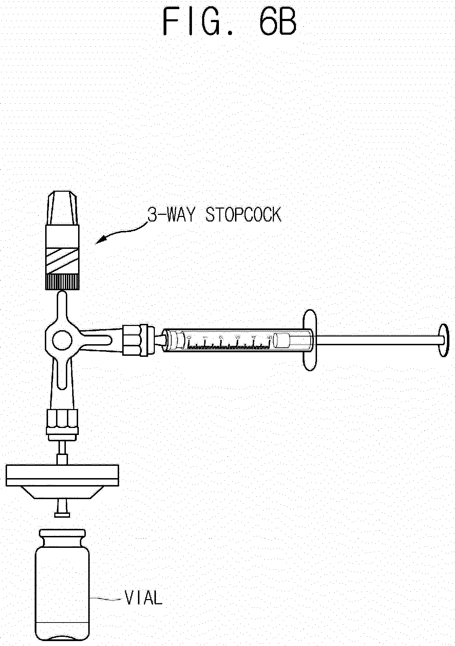

As explained above, the drug 25 for the disease treatment may be loaded to the micelle 20 and then be delivered to the human body. Detailed methods for identifying a drug loaded in the micelle will be explained in Figs. 6 to 10 below.

Figs. 6A and 6B show exemplary diagrams explaining a method for identifying a drug loaded to a micelle including a bubble for drug delivery in accordance with an example embodiment of the present invention.

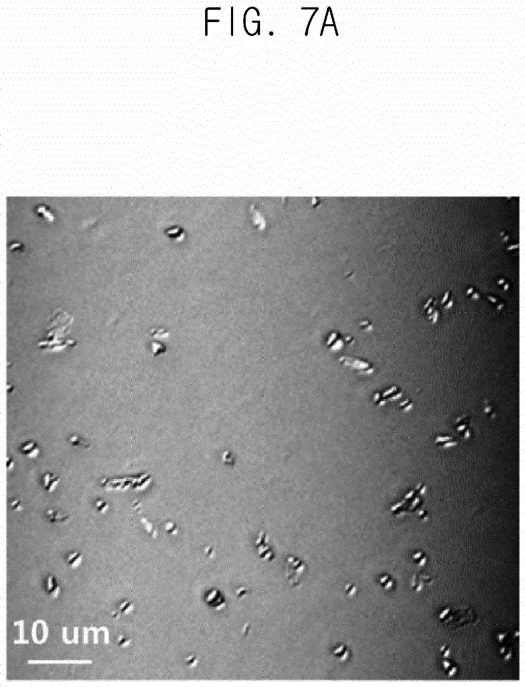

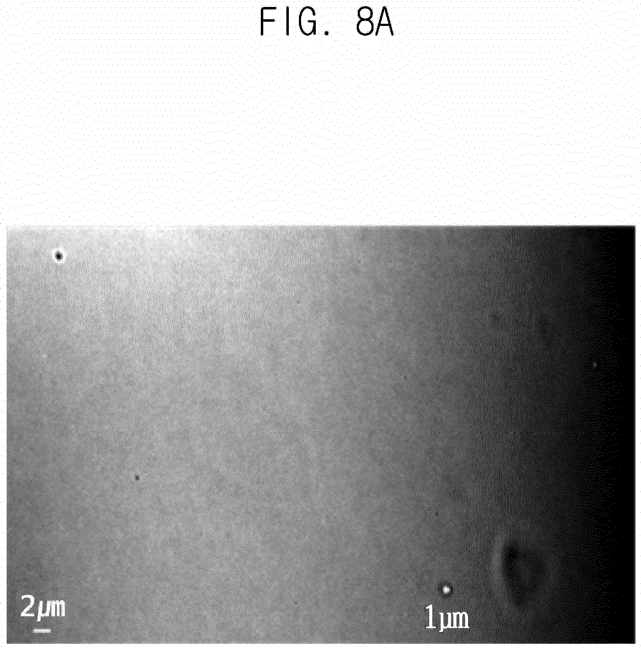

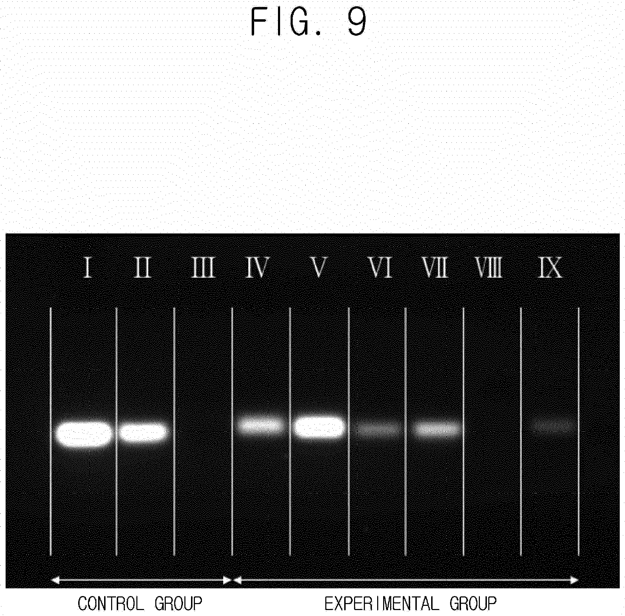

Besides, Figs. 7A to 7C illustrate exemplary diagrams in which a drug loaded to a micelle including a bubble for drug delivery is identified through confocal images in accordance with an example embodiment of the present invention; Figs. 8A to 8D depict exemplary diagrams in which a drug loaded to a micelle including a bubble for drug delivery is identified through microscopic images in accordance with an example embodiment of the present invention; and Fig. 9 is an exemplary diagram in which the drug loaded to a micelle including a bubble for drug delivery is identified through electrophoresis in accordance with an example embodiment of the present invention.

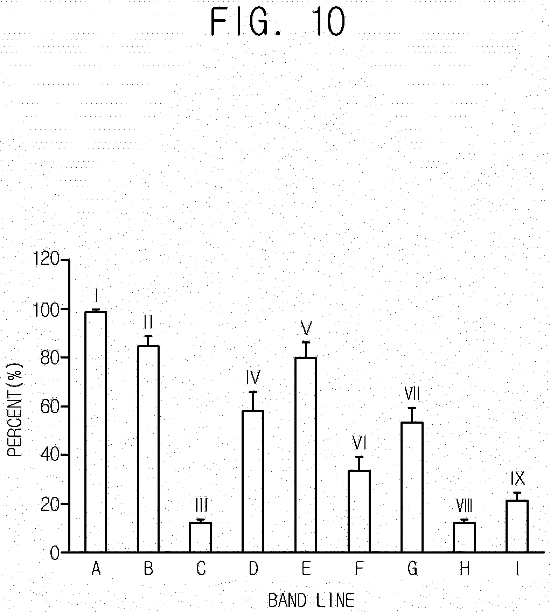

In addition, Fig. 10 is a graph representing a result identified through electrophoresis in accordance with an example embodiment of the present invention.

By referring to Fig. 6, a drug loaded in the micelle can be identified by removing siRNA from a solution, in which siRNA with a size of 3µm is mixed with a predetermined mixture, i.e., a mixture of micelles and perflouorobutane with a ratio of 1:100, through a vialmixer, in use of a 3-way stopcock, a syringe filter, and RNase as an enzyme cutting RNA.

For example, if a 100µm micelle is manufactured and a drug is loaded in the micelle, a syringe filter with a size of 10µm is installed and the syringe is compressed. Then, as the size of siRNA is smaller than that of the filter, the siRNA is filtered by a vial just as shown in Fig. 6A and only the micelle is left. At the time, if RNase is added and then extraction is performed with a 3-way stopcock as shown in Fig. 6B to remove the siRNA that might be left in the shell of the micelle, only the micelle from which the drug has been removed is left. It can be found out not only that the drug has been loaded to the micelle but also which kind of drug has been loaded through this.

As explained above, the micelle and the drug loaded in the micelle may be visually identified by using a variety of methods.

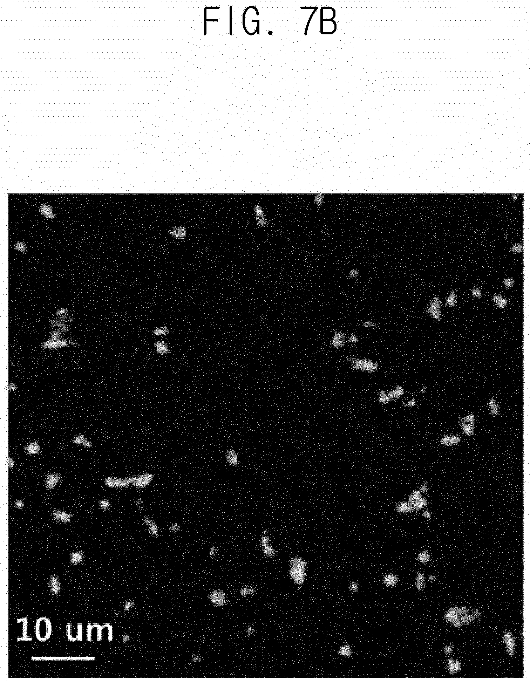

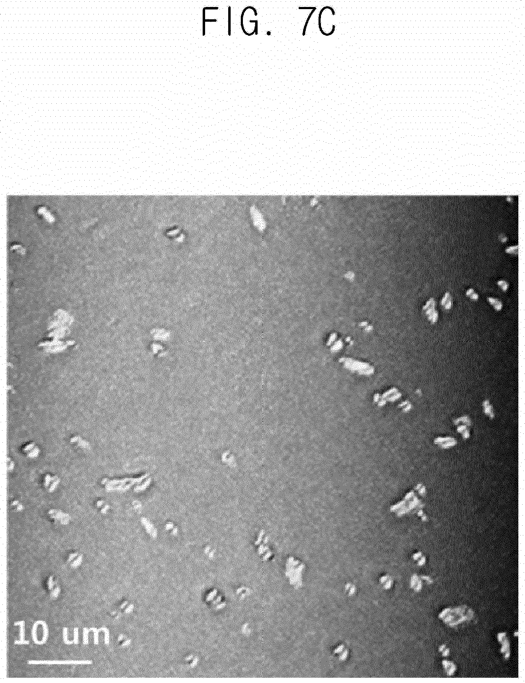

First of all, they can be identified through confocal images as shown in Fig. 7.

More specifically, Fig. 7A is an original image including multiple micelles A in different sizes and shapes and Fig. 7B is an image identified by fluorescently staining siRNA B with the wavelength of 488 nm. If the image of Fig. 7C, which is an image acquired by combining Fig. 7A and the Fig. 7B, is looked into, even though the RNA has been removed with the RNase, it can be found out that the position of siRNA B appearing in the fluorescent image is similar to that of the micelles A. Through this, it can be identified that the drug siRNA is protected by the micelle.

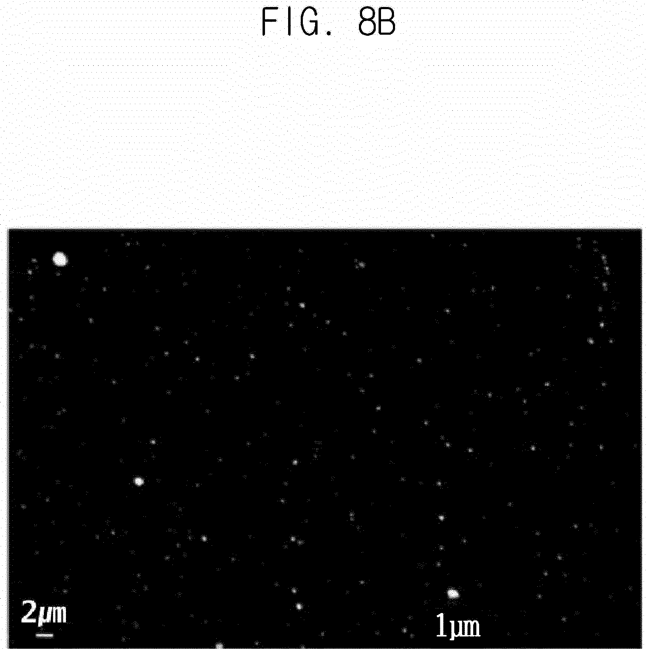

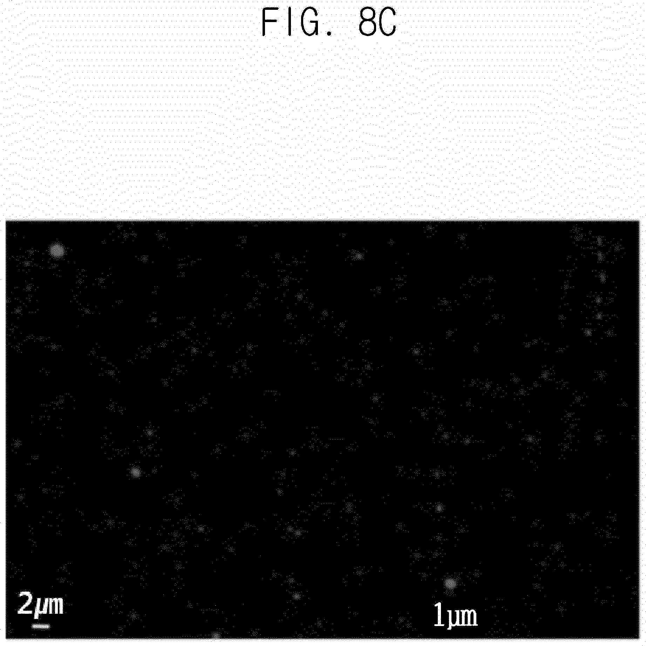

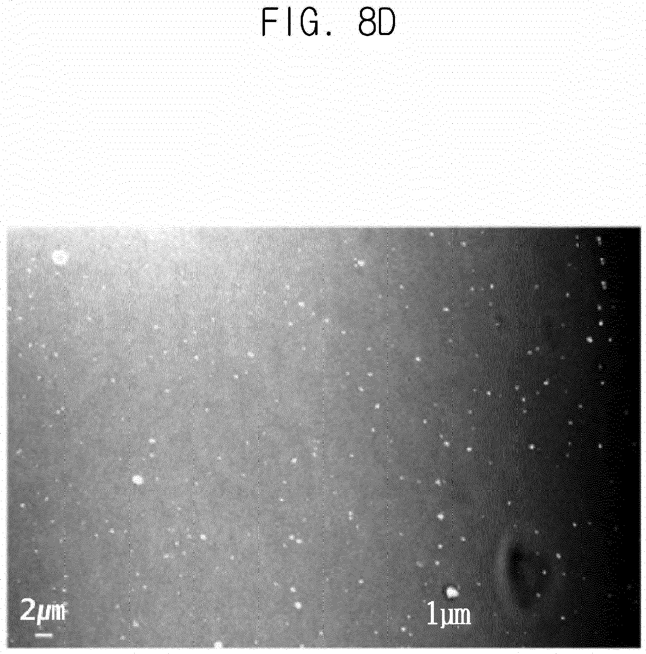

In addition, the micelle and the drug loaded in the micelle can be identified through microscopic images as illustrated in Fig. 8.

More specifically, Fig. 8A is an original image including multiple micelles in different sizes and shapes and Fig. 8B is an image visualized by fluorescently staining the lipid, i.e., substance of the shell of the micelle, with the wavelength of 534 nm. Besides, Fig. 8C is an image visualized by fluorescently staining siRNA as a drug contained in the micelle with the wavelength of 488 nm. If the image of Fig. 8D, which is an image acquired by combining Fig. 8B and Fig. 8C, is looked into, it can be found out that Fig. 8B depicting the image of the shell of the micelle and Fig. 8C representing the siRNA contained in the micelle, are overlapped approximately by 88%. Through this, it can be noticed that the drug contained in the micelle is capsuled and protected by the shell of the liposome.

Finally, the micelle and the drug loaded in the micelle can be identified through electrophoresis as illustrated in Fig. 9.

More preferentially, I in Fig. 9 is an image representing pure siRNA and II therein is an image showing a state of the pure siRNA to which the ultrasounds have been irradiated. Besides, III is an image illustrating a pure micelle, and IV is an image depicting a state of the micelle mixed with the siRNA. V is an image illustrating a state of the mixture of the micelle and the siRNA to which the ultrasounds have been irradiated while VI shows a state in which the shell is removed from the mixture of the micelle and the siRNA by using a filter and VII illustrates a state in which ultrasounds are irradiated after the shell has been removed from the mixture of the micelle and the siRNA by using the filter. Besides, VIII depicts a state in which RNase is added after the siRNA has been mixed with the micelle and IX is an image representing a state in which the ultrasounds are irradiated after RNase inhibitor is used to remove the RNase left after the RNase has been applied to the mixture of the micelle and the siRNA.

The respective results of electrophoresis as shown in Fig. 9 can be identified through graphs expressing percentages according to a band line as shown in Fig. 10. In comparison between a bar graph f illustrating the course of VI in Fig. 9 and a bar graph g representing the course of VII, if the ultrasounds are irradiated after the shell is removed from the micelle mixed with the siRNA by using the filter, it can be confirmed that the band line increases roughly by 20% because the siRNA having been protected by the shell gives an influence over the band signal due to the destruction of the shell of the micelle. Accordingly, it can be drawn that the siRNA is protected by the micelle through this.

Additionally, it can be confirmed from a bar graph h illustrating the course of VIII in Fig. 9 that the band signal barely appears due to the dissipation of the siRNA by the RNase because the siRNA has been mixed with the micelle and then the RNase has been added. Furthermore, it can be identified through a bar graph i depicting the course of IX in Fig. 9 that the band signal of approximately 20% is formed due to the siRNA left in the micelle.

Through this, it can be easily identified that the drug can be loaded in the micelle for treatment of diseases.

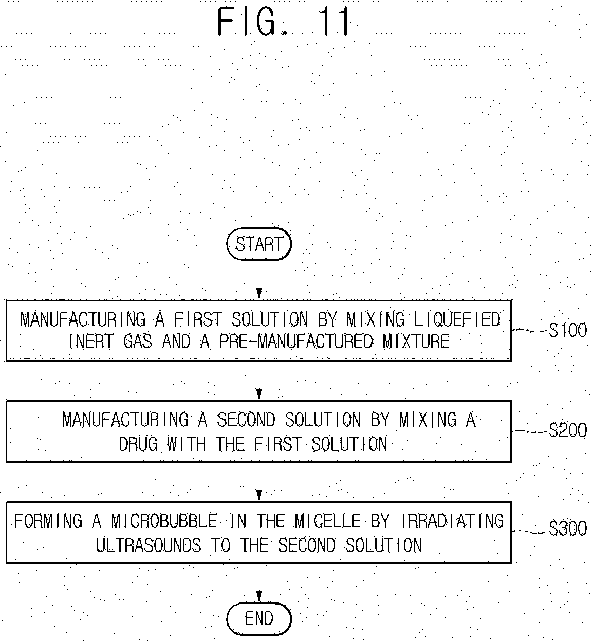

Fig. 11 is a flow chart explaining a method for manufacturing a micelle including a bubble for drug delivery in accordance with an example embodiment of the present invention.

By referring to Fig. 11, a method for manufacturing the micelle may include steps of: manufacturing a first solution by mixing liquefied inert gas and a pre-manufactured mixture at a step of S100; manufacturing a second solution by mixing a drug with the first solution at a step of S200; and forming a microbubble in the micelle by irradiating ultrasounds to the second solution at a step of S300.

More specifically, to manufacture the micelle, the first solution is manufactured by mixing the liquefied inert gas and the pre-manufactured mixture at a step of S100.

Herein, the pre-manufactured mixture may be manufactured by mixing at least one of saline solution, glycerol, propylene glycol, and powdered phospholipid.

For example, to manufacture the first solution, saline solution of 50 ml and glycerol of 2.5 ml are mixed first and then propylene glycol of 52.5 ml is mixed. Then, Dipalmitoyl Phosphatidyl Choline (DPPC) of 0.1 g and Diphenoxy Phosphoryl Azide (DPPA) of 0.01 g are mixed with the above-mentioned mixture including the saline solution, the glycerol and the propylene glycol; and then a revised mixture including the saline solution, the glycerol, the propylene glycol, DPPC and DPPA is heated through a hot plate at 60°C roughly for 30 minutes, thereby completing the manufacture of the "pre-manufactured mixture" mentioned at the step of S100. At the time, because DPPC and DPPA are powder like substances, the heating time through the hot plate may refer to a time when DPPC and DPPA are all melt but it is not limited to this. The time can be adjusted depending on the temperature.

After that, the pre-manufactured mixture of 2000 µl is extracted by using a micropipette and then kept in a vial of 2 ml. Then, the liquefied inert gas of 20 µl is mixed with the pre-manufactured mixture of 2000 µl kept in the vial of 2 ml by using the micropipette and then it is shaken for 45 seconds by using a Vialmix device.

Herein, perflouorobutane is used as the inert gas in that the boiling point of the perflouorobutane is -2.56°C and the perflouorobutane can be easily mixed with the pre-manufactured mixture. Accordingly, the liquefied perflouorobutane and the pre-manufactured mixture may be mixed together at the temperature range between -15°C and 5°C at which they can be easily mixed.

Thereafter, the first solution can be manufactured by filtering it (the mixture of the inert gas and the pre-manufactured mixture) in use of a membrane filter of 100 µm selected by referring to a bubble size.

Herein, the perflouorobutane is used as the inert gas but it is not limited to this.

Then, as explained above, the second solution may be manufactured by mixing a drug for diseases treatment with the first solution at a step of S200.

Accordingly, if the drug to be put into the first solution is changed, an appropriate drug applicable to treatment of at least one of diseases, e.g., cancers, skin diseases, brain diseases, and gene therapies, can be loaded to the micelle.

Therefore, the micelle which includes a microbubble can be created by irradiating the ultrasounds into the second solution to cause cavitation in the second solution at a step of S300.

Herein, the cavitation caused by ultrasounds means a phenomenon in which an empty space filled with gas is created in a liquid solution due to ultrasounds. In detail, the gas in the liquid gathers at a place at which the pressure is low in the liquid solution if the ultrasounds are irradiated into the liquid solution.

In other words, if the ultrasounds are irradiated to the second solution, the microbubble can be formed in the second solution due to the evaporation of the liquefied inert gas through the cavitation caused by the ultrasounds.

At the time, the drug left after the liquefied inert gas has been evaporated in the second solution is loaded at a liquid state in an intermediate layer between the microbubble and the shell and the shell may enclose the drug to allow the drug at the liquid state to be delivered safety to the human body by maintaining the intermediate layer at the liquid state.

As a result, the micelle may be formed to include a microbubble as a form of cavity and has a shape of a capsule enclosed by the shell and the drug can be safely delivered to the human body.

As explained above, in accordance with one example embodiment of the present invention, the micelle and a method for manufacturing the micelle can deliver the drug safely to the human body efficiently.

Besides, it is easily applicable to the treatment of all diseases because a drug to be loaded may be changed depending on a type of a disease.

Explanation has been made by referring to the desirable example embodiments of the present invention, but it could be understood that a person having ordinary skill in the technical field of the present invention may modify and change the present invention variously within the scope not exceeding the thought and area of the present invention described in the scope of the claims below.

## Claims

1. A micelle comprising a microbubble formed by irradiating ultrasounds to a solution into which liquefied inert gas and a drug for treatment of a disease are mixed together and evaporating the liquefied inert gas,
wherein the micelle comprises: the microbubble as a form of cavity, wherein the micelle is a unit structure enclosed by a shell, and wherein the micelle delivers the drug to a human body.

2. The micelle of Claim 1, wherein the unit structure is capsule-shaped.

3. The micelle of Claim 1, wherein the microbubble is a space formed by the evaporation of the liquefied inert gas in the solution due to cavitation caused by the ultrasounds.

4. The micelle of Claim 3, wherein the liquefied inert gas is perfluorobutane.

5. The micelle of Claim 4, wherein the solution is manufactured by mixing perfluorobutane and a pre-manufactured mixture in which at least one of saline solution, glycerol, propylene glycol, and powdered phospholipid at temperature range between -15°C and 5°C and then mixing the drug for the treatment of the disease.

6. The micelle of Claim 3, wherein the shell provides a space where the drug left in a liquid state after the evaporation of the liquefied inert gas.

7. The micelle of Claim 6, wherein the drug loaded to the unit structure is changeable to make the micelle applicable to treatment of at least one disease.

8. A method for manufacturing a micelle capable of delivering a drug, comprising the steps of:
manufacturing a first solution by mixing liquefied inert gas and a pre-manufactured mixture;
manufacturing a second solution by mixing the drug for treatment of a disease with the first solution; and
forming a microbubble in the micelle by irradiating ultrasounds to the second solution to create cavitation in the second solution.

9. The method of Claim 8, wherein the pre-manufactured mixture is manufactured by mixing at least one of saline solution, glycerol, propylene glycol, and powdered phospholipid.

10. The method of Claim 8, wherein, at the step of manufacturing the first solution, perflouorobutane as the liquefied inert gas is mixed with the pre-manufactured mixture at temperature range between -15°C and 5°C.

11. The method of Claim 8, wherein, at the step of manufacturing the second solution, the micelle is applicable to treatment of at least one disease by changing the drug to be mixed with the first solution.

12. The method of Claim 8, wherein, at the step of forming the microbubble in the micelle, the microbubble is formed by the evaporation of the liquefied inert gas in the second solution due to cavitation caused by the ultrasounds if the ultrasounds are irradiated to the second solution and the capsule-shaped micelle, which includes the microbubble as a form of cavity and is enclosed by a shell that provides a space, where the drug left in a liquid state after the evaporation of the liquefied inert gas, is formed.
